# EUROPÄISCHE PATENTANMELDUNG

(11) **EP 1 970 066 A1**
(43) Veröffentlichungstag der Anmeldung: **17.09.2008**
(21) Anmeldenummer: 07103925.9
(22) Anmeldetag: 12.03.2007
(51) Int. Cl.: A61K 33/00, A61K 9/28, A61K 9/30, A61K 9/20

(54) **Pharmazeutische Zusammensetzung zur Behandlung einer Übersäuerung des Blutes**

(71) Anmelder: Medice Arzneimittel Pütter GmbH & Co. KG, 58638 Iserlohn (DE)
(72) Erfinder: Kalisch, Peter., 58708 Menden (DE)
(74) Vertreter: Behnisch, Werner

(57) **Zusammenfassung**

Die vorliegende Erfindung betrifft eine pharmazeutische Zusammensetzung zur peroralen Verabreichung, die einen Arzneistoff und partikuläre Arzneistoffträger mit kontrollierter Arzneistofffreisetzung umfasst, wobei der Arzneistoff aus Erdalkali- oder Alkalimetallearbonaten ausgewählt ist. Die vorliegende Erfindung betrifft weiterhin die Verwendung dieser pharmazeutischen Zusammensetzung zur Behandlung einer metabolischen Azidose oder einer Übersäuerung des Blutes bei chronischer Niereninsuffizienz. Dies wird erreicht durch einen partikulären Arzneistoffträger, in dem der Arzneistoff in einer lipophilen Matrix eingebettet ist, wobei der Arzneistoffträger einen i-nagensaftresistenten Überzug aufweist

## Beschreibung

Die vorliegende Erfindung betrifft eine pharmazeutische Zusammensetzung zur peroralen Verabreichung, die einen Arzneistoff und partikuläre Arzneistoffträger mit kontrollierter Arzneistofffreisetzung umfasst, wobei der Arzneistoff aus Erdalkali- oder Alkalimetallcarbonaten ausgewählt ist. Die vorliegende Erfindung betrifft weiterhin die Verwendung dieser pharmazeutischen Zusammensetzung zur Behandlung einer metabolischen Azidose oder einer Übersäuerung des Blutes bei chronischer Niereninsuffizienz.

Unter einer Azidose wird ein Zustand der Übersäuerung des menschlichen oder tierischen Körpers verstanden. Liegt der pH-Wert im Blut unterhalb von 7,36 spricht man von einer Azidose. Der Normal-pH-Wert des Blutes liegt im Bereich von 7,36 bis 7,44. Bei darüberliegenden pH-Werten liegt eine Alkalose vor.

Unter einer metabolischen Azidose versteht man die Ansammlung einer zu großen Menge saurer Stoffwechselprodukten im Blut, wie beispielsweise bei Zuckererkrankung oder chronischer Nierenerkrankung. Der pH-Wert sinkt ab, wenn die Pufferkapazität des Blutpuffers gegenüber Säuren erschöpft sind. Dies führt zu einem plötzlichen starken Absinken des pH-Werts und zu einer akuten Übersäuerung. Es kann sich dabei oftmals um akut lebensbedrohliche Zustände handeln.

Die häufigsten Ursachen für eine metabolische Azidose sind Niereninsuffizienz oder ein isolierter tubulärer H⁺-Sekretionsdefekt der Nieren, eine diabetische Stoffwechselentgleisung, Urämie oder eine Vergiftung mit sauren Substanzen, wie beispielsweise Acetylsalicylsäure. Ein Patient mit metabolischer Azidose fällt vor allem durch seine verstärkte, tiefe und beschleunigte Atmung auf. Bei der Ketoazidose des Diabetikers ist unter Umständen ein charakteristischer Acetongeruch in der Atemluft zu bemerken. Entscheidend für die Erkennung und Quantifizierung der metabolischen Azidose ist eine Blutgasanalyse, wobei sich aus dem Basendefizit, dem pH-Wert und dem CO₂-Partialdruck leicht das Ausmaß der metabolischen Azidose und das Ausmaß der respiratorischen Kompensation des Körpers erkennen lässt.

Abgesehen von kausalen Therapien, wie beispielsweise der Gabe von Insulin bei der diabetischen Ketoazidose, besteht die Therapie bei der metabolischen Azidose bzw. der Übersäuerung des Blutes in der Gabe von basischen Substanzen, beispielsweise Natriumhydrogencarbonat oder anderen Puffersubstanzen, mit denen derartige Stoffwechselentgleisungen zumindest vorübergehend eingegrenzt werden können.

Entsprechende Medikamente sind bereits auf dem Arzneimittelmarkt verfügbar. Beispielhaft angeführt sei hier das Produkt Nephrotrans® der Firma Medice, Iserlohn, das pro Kapsel einen Gehalt von 500 mg Natriumhydrogencarbonat aufweist. Diese Kapseln sind magensaftresistent überzogen und lösen sich erst im Dünndarm auf. Dies führt zu einer Anhebung des Plasmahydrogencarbonatspiegels und zur Behebung eines Hydrocarbonatdefizites zur Pufferung einer zu hohen Wasserstoffionenkonzentration und zu einer Steigerung des Blut-pH bei metabolischer Azidose.

Eine Freisetzung von Natriumhydrogencarbonat im Magen muss unbedingt vermieden werden, da Hydrogencarbonat durch das in der Magensäure enthaltene HC1 zu Natriumchlorid und Kohlendioxidgas umgewandelt wird und somit für jedwede therapeutische Anwendung zur Anhebung des Blut-pH nicht mehr zur Verfügung steht. Insbesondere können sich Probleme bei postprandialer Einnahme des Arzneimittels ergeben, da dann, abhängig von der zugeführten Nahrung, der pH des Magens bis auf Werte von sogar pH 6,0 ansteigen kann, woraus sich ein verfrühter Abbau der magensaftresistenten Beschichtung ergeben kann. Hierdurch kann sich bei Ulcus-Patienten durch das entstehende Kohlendioxidgas sogar die Gefahr einer Ruptur ergeben.

Das oben genannte Präparat Nephrotrans® weist eine besondere Galenik auf, die Natriumhydrogendcarbonat direkt an den physiologischen Resorptionsort im Dünndarm transportiert. Im Produkt Nephrotrans® ist das Natriumhydrogencarbonat in einer Mischung fetthaltiger Materialien enthalten, wobei die fetthaltigen Materialien eine pastöse Form aufweisen. Diese pastöse Zubereitung ist in Weichgelatinekapseln enthalten, die ihrerseits magensaftresistent überzogen sind. Nach Verabreichung wird nach Passage des Magens und Auflösung der magensaftresistenten Beschichtung ein langsames Herauslösen von Natriumhydrogencarbonat erfolgen.

Es handelt sich bei Nephrotrans® um eine monolithische Darreichungsform, wobei sich die Gefahr des sogenannten "dose dumpings" ergibt. Nach den Definition der Food & Drug Administration bedeutet der Begriff "dose dumping", dass eine Dosierungsform in unerwünschter Weise eine zu große Menge des Arzneistoffes freisetzt. Dieser Effekt betrifft insbesondere Arzneimittel mit verzögerter Freisetzung. Beispielsweise kann sich insbesondere bei postprandialer Einnahme eine Situation ergeben, in der eine monolithische Darreichungsform im Magen akkumuliert (d.h. nicht dazu in der Lage ist, in angemessener Zeit den Pylorus zu durchqueren und in den Zwöffingerdarm etc. überzutreten). Bei einem zeitverzögerten Übertritt in den Darm kann es dann zu einer übermäßigen Freisetzung des Wirksstoffes, also zu einem dose dumping kommen.

Es besteht also nach wie vor ein Bedarf nach maßgeschneiderten und sicheren Arzneimitteln, die zur Behandlung der metabolischen Azidose bzw. der Übersäuerung des Blutes eingesetzt werden können.

Es ist somit eine Aufgabe der vorliegenden Erfindung, eine Dosierungsform zur peroralen Verabreichung bereitzustellen, die eine gezielte, kontrollierte Zufuhr und Abgabe von Erdalkali- oder Alkalimetallcarbonaten im Dünndarm gewährleistet und somit zur Einstellung des Blut-pH verwendet werden kann und gleichzeitig die Gefahr eines "dose dumpings" verhindert.

Diese Aufgabe wird durch den Gegenstand der unabhängigen Ansprüche gelöst. Bevorzugte Ausführungsformen ergeben sich aus den Unteransprüchen.

Gemäß eines ersten Aspektes betrifft die vorliegende Erfindung eine pharmazeutische Zusammensetzung zur peroralen Verabreichung, die einen Arzneistoff und partikuläre Arzneistoffträger mit kontrollierter Arzneistofffreisetzung umfasst, wobei der Arzneistoff ein Erdalkalimetall- oder Alkalimetallcarbonat ist und in dem partikulären Arzneistoffträger in einer lipophilen Matrix eingebettet ist, und wobei der Arzneistoffträger einen magensaftresistenten Überzug aufweist.

Durch Bereitstellung einer pharmazeutischen Darreichungsform mit einem (multi)partikulären Arzneistoffträger kann die oben genannte Gefahr des dose dumpings, die bei monolithischen Darreichungsformen auftritt, ausgeschlossen werden.

Das Wirkprinzip der pharmazeutischen Zusammensetzung der vorliegenden Erfindung ist zunächst eine Freisetzungsverzögerung im sauren Milieu (Magen), das der partikuläre Arzneistoffträger im wesentlichen unverändert durchlaufen muss, wonach bei alkalischem pH und Auflösung der magensaftresistenten Beschichtung die pharmazeutische Zusammensetzung in Form vieler einzelner partikulärer Arzneistoffträger im Dünndarm vorliegt. Hier werden dann in der Folge der oder die Wirkstoffe durch Diffusion bzw. langsames Herauslösen aus der lipophilen Matrix freigesetzt. Hierdurch ergibt sich ein gleichmäßiges Freisetzungsverhalten durch das, wie oben angesprochen, die Gefahr eines dose dumpings ausgeschaltet wird.

Das Freisetzungsverhalten entspricht und wird durchgeführt unter den im Europäischen Arzneibuch (Ph. Eur.) angegebenen Vorschriften, siehe insbesondere 2.9.1-2.9.3 sowie die einschlägige Kommentierung.

Zusätzlich kann die Verwendung partikulärer Arzneistoffträger, insbesondere im Falle einer postprandialen Einnahme, den Vorteil mit sich bringen, dass diese aufgrund ihrer geringeren Größe als eine monolithische Darreichungsform, deutlich früher den Pylorus durchdringen und zu einer gleichmäßigeren Freisetzung auch unter diesen Umständen führen können.

Der Begriff "partikulär" ist im Gegensatz zu den üblichen monolithischen Darreichungsformen zu sehen, d.h., die pharmazeutische Zusammensetzung der vorliegenden Erfindung besteht aus einer Vielzahl von getrennten Einzelpartikeln. Diese Einzelpartikel enthalten jeweils eine definierte Dosis des Arzneistoffes. Der sich daraus ergebende Vorteil ist neben den oben genannten eine deutlich größere Gesamtoberfläche der Darreichungsform.

Zu erwähnen ist zudem, dass der Arzneistoffträger den Wirkstoff langsam freisetzen kann, was ein wesentlicher Unterschied zu einer Tablette ist.

In einer bevorzugten Ausführungsform weist die pharmazeutische Zusammensetzung eine Form auf, in der der partikuläre Arzneistoffträger in Einzeldosen- oder Mehrdosenbehältnissen vorliegt. Als in der Technik bekannte Einzeldosisbehältnis bietet sich hier insbesondere die Form einer Kapsel an. Insbesondere weist die Kapsel die Form einer Hartkapsel auf. Es können hierbei alle üblichen Formen von Hartkapseln verwendet werden, die beispielsweise mit geläufigen Coni-Snap^{®} oder Snap-Fit^{®} -Verschlüssen ausgestattet sind. Abhängig von der verwendeten Wirkstoffdosis können die geläufigen Kapselgrößen von 00 bis 2 verwendet werden.

Der partikuläre Arzneistoff weist einen durchschnittlichen Teilchendurchmesser von ca. 100 bis 4.000 µm (0,1 bis 4 mm) auf. Beispielsweise können Pellets verwendet werden, die einen Teilchendurchmesser von ca. 200-300 mm aufweisen.

Auch können Mikrotabletten verwendet werden, die einen Durchmesser von 2 bis max. 4 mm aufweisen können. Hierzu ist zu beachten, daß es bei der Herstellung der Mikrotabletten zu einer starken Erwärmung kommen kann. Es müssen dann ev. spezielle, d.h. wärmebeständigere, lipophile Matrixbestandteile gewählt werden und die Matrizentischchen müssen gekühlt werden.

Sollte der partikuläre Arzneistoffträger in Einzeldosisbehältnissen, wie oben angesprochen, abgefüllt werden, weist eine solche Einzeldosis in der Regel eine Menge von 200 bis 1.500 mg des Erdalkali- oder Alkalimetallcarbonatsalzes auf. Als Erdalkalimetall- oder Alkalimetallcarbonate kommen insbesondere Natriumcarbonat, Magnesiumcarbonat, Magnesiumhydrogencarbonat, Calciumhydrogencarbonat, Natriumhydrogencarbonat und Calciumcarbonat sowie Mischungen hiervon in Betracht. Natriumhydrogencarbonat ist besonders bevorzugt. Eine Einzeldosis umfasst hier besonders bevorzugt 300 bis 700 mg Natriumhydrogencarbonat.

Es sei darauf hingewiesen, dass der Begriff Erdalkalimetall- bzw. Alkalimetallcarbonate gemäß der oben angesprochenen Definition nicht nur Carbonate selbst, sondern auch Hydrogencarbonate umfasst.

Die pharmazeutische Zusammensetzung kann neben einem Einzeldosisbehältnis auch in Form von Mehrdosenbehältnissen vorliegen, wobei letztere vorzugsweise die Form von Beuteln oder Sachets aufweisen.

In Mehrdosenbehältnissen, wie z. B. Beuteln oder Sachets können die partikulären Arzneistoffträger mit einem geeigneten Dosierungsinstrument, wie beispielsweise einem Messlöffel, direkt zum Verbrauch ausgemessen werden.

Wie bereits oben angesprochen weisen die Arzneistoffträger der vorliegenden Erfindung einen magensaftresistenten Überzug auf. Es können hier die in der Technik bekannten magensaftresistenten Beschichtungen gewählt werden, die beispielsweise aus Celluloseacetatphthalat, Hydroxypropylmethylcellulosephthalat, Hydroxypropylmethylcellulosesuccinat, Carboxymethylethylcellulose, Poly(vinylacetatphthalat), Poly([meth]acrylsäureco[meth]-acrylsäuremethylester) ausgewählt sind. Es können jedoch auch alle anderen magensaftresistente Filme bildenden Ausgangsmaterialien verwendet werden, insofern sie den Freisetzungsvorschriften des europäischen Arzneibuchs entsprechen.

Die partikulären Arzneistoffträger der vorliegenden Erfindung bestehen zu einem großen Teil aus einer lipophilen Matrix, in der die Erdalkalimetall- oder Alkalimetallcarbonatsalze eingebettet sind. Solche lipophilen Matrixmaterialien bestehen typischerweise aus klassischen hydrophoben Substanzen, wie beispielsweise Triglyceriden (beispielsweise Vitokan^{®}) oder auch ähnlichen Substanzen, die eine ausreichende Diffusionsbarriere im Dünndarm bereitstellen. Hier sind beispielsweise Diglyceride, Glycerinmonostearat, Paraffine, Wachse anzusprechen. Der Fachmann ist auf Grundlage der speziellen Anforderungen des Einzelfalls und aufgrund seines Fachwissens dazu in der Lage, die jeweils geeigneten lipophilen Matrixsubstanzen zu bestimmen und dem Zweck entsprechend einzusetzen. Beispielsweise wird sich bei der Herstellung von Mikrotabletten (siehe oben), bei denen gegebenenfalls mit einer hohen Wärmebelastung zu rechnen ist, die Verwendung längerkettiger Paraffine mit höherem Schmelzpunkt anbieten.

Das Gewichtsverhältnis des lipophilen Matrixbestandteils zu dem eingesetzten Arzneistoff im partikulären Arzneistoffträger kann abhängig von den Gegebenheiten des Einzelfalls schwanken. Ein Gewichtsverhältnis von 30:70 bis 70:30, z.B. auch 50:50, ist jedoch als durchaus realistisch einzustufen.

Bezüglich weiterer Informationen zu Matrixmaterialien, die im Kontext der vorliegenden Erfindung zur Herstellung von partikulären Arzneistoffträgern verwendet werden können, wird auf die einschlägige Fachliteratur verwiesen, insbesondere ...

Bauer/Frömming/Führer "Lehrbuch der Pharmazeutischen Technologie" (Wissenschaftliche Verlagsges.; Auflage: 8., durchges. u. aktualis. Aufl. (März 2006)).

Darüber hinaus kann der partikuläre Arzneistoffträger übliche Formen von Granulaten oder, wie oben angesprochen, abgerundet als Pellets, oder einer Mikrotablette aufweisen.

Die vorliegende pharmazeutische Zusammensetzung wird zur Behandlung der metabolischen Azidose und/oder einer Übersäuerung des Blutes bei chronischer Niereninsuffizienz verwendet.

Die vorliegende Erfindung wird nunmehr anhand einer Abbildung sowie der beigefügten Beispiele veranschaulicht werden.

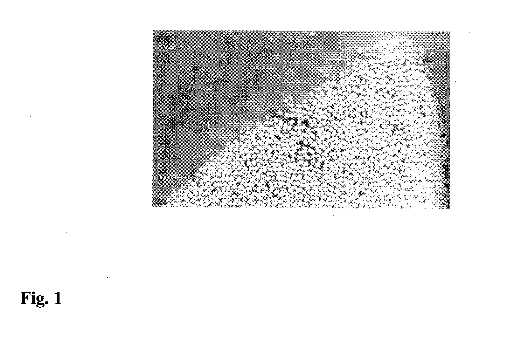

Figur 1 zeigt partikuläre Arzneistoffträger der Erfindung, die einen Durchmesser von ca. 1000 bis 3000 µm aufweisen. Der Triglyceridanteil in den Arzneistoffträgern beträgt ca. 50%.

### Beispiele:

### Equipment

| | |
|---|---|
| Abwiegung: | Analysenwaage |
| Mischen: | Labormischer LM 20 (Bohle, Ennigerloh) |
| Pulverdosierung: | KT20 (K-Tron, CH-Niederlenz) |
| Extrusion: | Zweischneckenextruder Mikro 27GL-28D (Leistritz, Nürnberg) mit Düsenplatte (1 mm Lochdurchmesser, 23 Bohrungen) |
| Rundung: | Spheronizer RM300 (Schlüter, Neustadt) |

### Hergestellte Chargen

| | |
|---|---|
| E20070202a | 400 g Witocan 42/44 Pulver (Sasol, Witten), Ch. 502021 400 g Natriumhydrogencarbonat (Merck, Darmstadt), Ch. K36651523 645 |
| E20070202b | 700 g Witocan 42/44 Pulver (Sasol, Witten), Ch. 502021 300 g Natriumhydrogencarbonat (Merck, Darmstadt), Ch. K36651523 645 |
| E20070202c | 160 g Witocan 42/44 Pulver (Sasol, Witten), Ch. 502021 640 g Natriumhydrogencarbonat (Merck, Darmstadt), Ch. K36651523 645 |
| E20070202b2 | 700 g Witocan 42/44 Pulver (Sasol, Witten), Ch. 502021 300 g Natriumhydrogencarbonat (Merck, Darmstadt), Ch. K36651523 645 |

### Versuchsdurchführung und -bedingungen

Die beiden Ausgangsstoffe (Hilfsstoff: pulverförmiges Triglycerid Witocan und Arzneistoff: Natriumhydrogencarbonat) wurden im Labormischer für 10 min bei 25 U/min gemischt. Über den Pulverdosierer wurde die Mischung dem Zweischneckenextruder zugeführt und unter den aufgeführten Bedingungen extrudiert. Es wurden zylinderförmige Extrudate erhalten, die in der Rundungsmaschine bei den angegebenen Bedingungen gebrochen und zu sphärischen bis ellipsoiden Partikeln ausgerundet wurden.

| | |
|---|---|
| E20070202a | Extruder: Förderrate 40 g /min bei 50 U/min, 6 Segmente bei 25 °C, letztes Segment bei 15 °C Spheronizer: etwa 300 g, 5 min, bei 1000 U/min: 33-34 °C |
| E20070202b | Extruder: Förderrate 40 g /min bei 50 U/min, alle Segmente bei 25 °C Spheronizer: etwa 300 g, 5 min bei 1000 U/min: 33-34 °C, 5 min bei 1500 U/min: 31-32°C |
| E20070202c | Extruder: Förderrate 40 g /min bei 50 U/min, alle Segmente bei 25 °C Spheronizer: etwa 300 g, 5 min bei 1000 U/min: 33-34 °C |
| E20070202b2 | Extruder: Förderrate 40 g /min bei 50 U/min, alle Segmente bei 30 °C; nicht gerundet |

### Versuchsergebnis

Bei allen Extrusionsversuchen wurden zylinderförmige Extrudate erhalten, die ein mehr oder weniger homogenes Erscheinungsbild besitzen. Die höheren Anteile an Triglycerid in E20070202a (50 %), E20070202b und E20070202b2 (70%) zeigen gegenüber E20070202c (20 %) keinen Vorteil. Das Extrudat mit dem niedrigsten Triglyceridanteil zeigt allerdings ungleichmäßigere Extrudate und neigt zur Blockierung der Düsenöffnungen. Bei Erhöhung des Druckes oder der Temperatur ergeben sich durch das Durchschmelzen des Triglcerids gekräuselte Extrudate und z.T. große, aus der Schmelze gebildete Platten außerhalb des Extruders als Abfallprodukte.

Beim Runden entstehen unter den konstant gehaltenen Versuchsbedingungen keine exakt runden Partikel, sondern zylinderförmige Granulate von ca. 1 mm Durchmesser und bis zu 3 mm Länge (siehe Fig. 1).

| Ausbeuten: | | | |
|---|---|---|---|
| | Unterkorn (<850µm) | Oberkorn (>2000µm) | Ausbeute (850µm-2mm) |
| E20070202a | 1,7% | <0,1 % | 88,0 % |
| E20070202b | 0,5% | 22,2 % | 62,6 % |
| E20070202c | 5,8% | 11,8% | 71,8% |

E20070202b2 wurde nicht sphäronisiert. Daher erfolgte auch keine Kornklassifikation.

Es ergab sich kein Vorteil der Temperatureinstellung der Heizsegmente auf 30 °C statt ansonsten 25 °C. Wegen des Auftretens von großen Aggregaten ist eine geringere Ausbeute zu erwarten.

### Schlußfolgerung:

Natriumbicarbonat lässt sich mit dem Triglycerid Witocan 42/44 extrudieren und zu ellipsoiden Partikeln anrunden. Temperatur- und/oder Druckerhöhung bei der Extrusion erbringt keine Vorteile. Diese Untersuchungen sollten allerdings mit drastischeren Unterschieden in den Prozessparametern wiederholt werden. Außerdem sollten andere Qualitäten von Triglyceriden, Mischungen von Triglyceriden und Zumischungen anderer Substanzen getestet werden, um optimale Pellets zu erhalten.

## Patentansprüche

1. Pharmazeutische Zusammensetzung zur peroralen Verabreichung, die einen Arzneistoff und partikuläre Arzneistoffträger mit kontrollierter Arzneistofffreisetzung umfasst,
**dadurch gekennzeichnet,**
**dass** der Arzneistoff ein Erdalkalimetall- oder Alkalimetallcarbonat ist, wobei der Arzneistoff in dem partikulären Arzneistoffträger in einer lipophilen Matrix eingebettet ist und wobei der Arzneistoffträger einen magensaftresistenten Überzug aufweist.

2. Pharmazeutische Zusammensetzung nach Anspruch 1, wobei das Erdalkalimetall-oder Alkalimetallcarbonat aus Natriumcarbonat, Magnesiumcarbonat, Magnesiumhydrogencarbonat, Calciumhydrogencarbonat, Natriumhydrogencarbonat und Calciumcarbonat, sowie Mischungen hiervon ausgewählt ist.

3. Pharmazeutische Zusammensetzung nach Anspruch 2, wobei das Alkalimetallcarbonat Natriumhydrogencarbonat ist.

4. Pharmazeutische Zusammensetzung nach einem oder mehreren der Ansprüche 1-3, wobei der partikuläre Arzneistoffträger in Einzeldosen- oder Mehrdosenbehältnissen vorliegt.

5. Pharmazeutische Zusammensetzung nach einem oder mehreren der vorhergehenden Ansprüche, wobei der partikuläre Arzneistoffträger einen mittleren Teilchendurchmesser von 100-4.000 µm aufweist.

6. Pharmazeutische Zusammensetzung nach einem oder mehreren der vorhergehenden Ansprüche, wobei der partikuläre Arzneistoffträger in einem Einzeldosenbehältnis vorliegt, das die Form einer Kapsel aufweist.

7. Pharmazeutische Zusammensetzung nach Anspruch 6, wobei die Kapsel eine Hartkapsel ist.

8. Pharmazeutische Zusammensetzung nach Anspruch 1-7, wobei das Einzeldosisbehältnis eine Menge von 200-1.500 mg Natriumhydrogencarbonat aufweist.

9. Pharmazeutische Zusammensetzung nach Anspruch 8, wobei das Einzeldosisbehältnis eine Menge von 300-700 mg Natriumhydrogencarbonat aufweist.

10. Pharmazeutische Zusammensetzung nach einem oder mehreren der Ansprüche 1-9, wobei der partikuläre Arzneistoffträger als Mehrdosenbehältnis vorliegt, das die Form eines Beutels oder Sachets aufweist.

11. Pharmazeutische Zusammensetzung nach einem oder mehreren der vorhergehenden Ansprüche, wobei der magensaftresistente Überzug aus Celluloseacetatphthalat, Hydroxypropylmethylcellulosephthalat, Hydroxypropylmethylcellulosesuccinat, Carboxymethylethylcellulose, Poly(vinylacetatphthalat), und/oder Poly([meth]acryl-säureco[meth]-acrylsäure-methylester) ausgewählt ist.

12. Pharmazeutische Zusammensetzung nach einem oder mehreren der vorhergehenden Ansprüche, wobei das lipophile Matrixmaterial aus Triglyceriden, Diglyceriden, Glycerinmonostearat, Paraffinen und/oder Wachsen ausgewählt ist.

13. Pharmazeutische Zusammensetzung nach einem oder mehreren der vorhergehenden Ansprüche, wobei der partikuläre Arzneistoffträger in Form von Pellets, Mikrotabletten oder Granulaten vorliegt.

14. Verwendung einer pharmazeutischen Zusammensetzung nach einem der Ansprüche 1-13 zur Behandlung einer metabolischen Azidose und/oder einer Übersäuerung des Blutes bei chronischer Niereninsuffizienz.
